# EUROPEAN PATENT SPECIFICATION

(11) **EP 4 356 961 B1**
(45) Date of publication and mention of the grant of the patent: **14.10.2026**
(21) Application number: 24156914.4
(22) Date of filing: 11.06.2020
(51) Int. Cl.: A61K 38/01, A61K 38/38, A61K 35/20, A23C 21/00, A23L 33/19, A61K 9/51, A61K 45/06, A61P 3/04, A61P 3/08, A61P 3/10, A23L 33/00, A61K 9/00, A61K 9/107

(54) **USE OF WHEY PROTEIN MICELLES FOR CONTROLLING POSTPRANDIAL GLUCOSE RESPONSE**
VERWENDUNG VON MOLKEPROTEINMIZELLEN ZUR KONTROLLE DER POSTPRANDIALEN GLUKOSEREAKTION
UTILISATION DE MICELLES DE PROTÉINES DE LACTOSÉRUM POUR RÉGULER LA RÉPONSE AU GLUCOSE POSTPRANDIAL

(30) Priority: 13.06.2019 EP 19180009
(43) Date of publication of application: 24.04.2024
(62) Document number(s) of the earlier application(s) in accordance with Art. 76 EPC: 20731864.3 / 3 982 992
(73) Proprietor: Société des Produits Nestlé S.A., 1800 Vevey (CH)
(72) Inventor: BOVETTO, Lionel Jean René, 1522 Lucens (CH); DARIMONT-NICOLAU, Christian, 1012 LAUSANNE (CH); EGLI, Léonie, 1807 Blonay (CH); RYTZ, Andreas, 1084 CARROUGE (CH)
(74) Representative: Gordon, Kirsteen Helen

(56) References cited:
- US-A1- 2014 287 056
- TINA AKHAVAN ET AL: "Effect of premeal consumption of whey protein and its hydrolysate on food intake and postmeal glycemia and insulin responses in young adults", THE AMERICAN JOURNAL OF CLINICAL NUTRITION, vol. 91, no. 4, 17 February 2010 (2010-02-17), US, pages 966 - 975, XP055646645, ISSN: 0002-9165, DOI: 10.3945/ajcn.2009.28406
- DANIELA JAKUBOWICZ ET AL: "Incretin, insulinotropic and glucose-lowering effects of whey protein pre-load in type 2 diabetes: a randomised clinical trial", DIABETOLOGIA, vol. 57, no. 9, 10 July 2014 (2014-07-10), BERLIN, DE, pages 1807 - 1811, XP055351543, ISSN: 0012-186X, DOI: 10.1007/s00125-014-3305-x
- JING MA ET AL: "Sustained effects of a protein 'preload' on glycaemia and gastric emptying over 4 weeks in patients with type 2 diabetes: A randomized clinical trial", DIABETES RESEARCH AND CLINICAL PRACTICE, vol. 108, no. 2, 25 February 2015 (2015-02-25), NL, pages e31 - e34, XP055646648, ISSN: 0168-8227, DOI: 10.1016/j.diabres.2015.02.019

## Description

The present invention generally relates to the use of whey protein micelles (WPM), administered before subsequent administration of a meal, to decrease postprandial glucose (PPG) response. The invention also relates to whey protein micelles, administered before subsequent administration of a meal, for use in a non-therapeutic condition to an increase in postprandial glucose in a subject.

### BACKGROUND

Overweight and obesity are defined as abnormal or excessive fat accumulation that presents a risk to health. An "overweight" human has a body mass index (BMI) between 25 and 30 kg/m2 whereas an "obese" human has a BMI of at least 30 kg/m2, for example 30-39.9 kg/m2.

Obesity is a chronic metabolic condition that has reached epidemic proportions in many areas of the world. Obesity is the major risk factor for serious co-morbidities such as type 2 diabetes mellitus, cardiovascular disease, dyslipidaemia and certain types of cancer. Overweight and obesity typically are a result of excessive accumulation of energy from carbohydrate, fat and the like. The additional weight is typically retained in the form of fat under the skin or around the viscera.

There is a relationship between high glucose levels post-prandially after a meal and a slow return to baseline glucose levels increase the individual's risk of becoming glucose intolerant or pre-diabetic. Overweight and obese individuals are particularly at risk. Diabetes is a metabolic condition characterized primarily by high blood glucose levels that result from the body's inability to make or use insulin. Hyperglycemia can lead to numerous clinical complications including blindness, limb amputations, heart attack or stroke.

The most common types of diabetes are insulin-dependent diabetes (Type-1 diabetes T1D) and non-insulin-dependent diabetes (Type-2 diabetes T2D). T2D is by far the most abundant type, and the increase in Type-2 diabetes (T2D) is mainly driven by increasing obesity rates.

Additionally, pre-diabetic conditions, defined as having a blood glucose higher than normal but not high enough to be diagnosed as diabetic, are contributing significantly to the strong rise of the diabetic population.

Insulin resistance (a low insulin sensitivity) occurs also in pregnant subjects. This is due to hormonal changes that help to ensure the transfer of nutrients from the pregnant subject to the fetus. As described above, in response to insulin resistance the pancreas may secrete more insulin to compensate. These subjects are considered as having an impaired glucose tolerance (hereinafter IGT). Eventually the pancreas may fail to keep up with the body's increased need for insulin, leading to type-2 diabetes. Any degree of glucose intolerance with onset or first recognition during pregnancy is referred to as Gestational Diabetes Mellitus (GDM).

The pathophysiology of the development of Type-2 diabetes is complex and multifactorial. Obesity, sedentary life style, and/or increased age may lead to insulin resistance and to increased circulating insulin concentrations over time. At some point, a loss of control of blood glucose begins to emerge, resulting in impaired glucose tolerance (IGT) or impaired fasting glucose (IFG) and may ultimately result in Type-2 diabetes. Therefore, IGT and IFG refer to metabolic states intermediate between normal glucose homeostasis and diabetes.

A further test, the oral glucose tolerance test (OGTT), may be performed to assess whether the patient is diabetic or has IGT. The OGTT consists of a glucose drink containing 75 g of glucose. The patient's blood sugar level is measured at one and two hours following administration of the drink.

Glucose is an essential nutrient for the human body, so its circulating levels must be carefully maintained constant in order to supply adequate amounts to peripheral tissues. The liver plays a central role in glucose homeostasis by balancing uptake and storage of glucose via glycogenesis and its release via glycogenolysis and gluconeogenesis. An impairment of glucose homeostasis is a typical feature of Type-2 diabetes. Patients with Type-2 diabetes exhibit increased hepatic glucose production (HGP), which is identified as the main cause of fasting hyperglycemia and is associated with a reduced plasma glucose clearance (Gastaldelli A, et al., Diabetes 2000; 49:1367-1373), and also a 25-45% reduced synthesis of glycogen compared with non-diabetic subjects (Roden M, et al., Best Pract Res Clin Endocrinol Metab. 2003; 17:365-83).

Optimal glycemic control is fundamental to the management of diabetes. Both fasting plasma glucose (FPG) and postprandial plasma glucose (PPG) levels correlate with the risk of complications and contribute to the measured glycated hemoglobin (A1C) value. A1C levels >7.0% are associated with a significantly increase risk of both microvascular and cardiovascular (CV) complications. PPG is an important component of overall hyperglycemia and may be the predominant component in patients who are closer to A1C goal and in older adults.

PPG are determined by several factors, such as the total caloric value of a meal, macronutrient composition, and carbohydrate quality (e.g., glycemic index/load), all of which may be monitored and controlled. However, multiple other factors involved in diseases such as T2D are more complex because they cannot be controlled and are variable between individuals. These include gastric emptying rate, intestinal absorption rate, enteroendocrine incretin secretion, incretin sensitivity, pancreatic beta-cell insulin secretory function, hepatic insulin extraction, hepatic glucose production, glucose effectiveness, glucose uptake in all tissues (especially brain, adipose, liver, muscle), insulin sensitivity, and renal glucose reabsorption

Limiting blood glucose peaks after a meal in diabetic subjects constitutes an important target of the overall glycemic control strategy. Uncontrolled PPG is common in diabetes. It contributes to overall hyperglycemia and is associated with poor outcomes. Treatment options that specifically target PPG are, therefore, critical components to achieving and sustaining glycemic control in patients with Type-1 diabetes (T1D) and Type-2 diabetes (T2D), and might prevent pre-diabetic subjects to advance to a diagnosed diabetic condition.

Evidence is importance of post-prandial glucose in both the management and prevention of type 2 diabetes. Post-prandial glucose was shown to be the main contributor to total glucose fluctuations in type 2 diabetes patients with HbA1c <8%, i.e. well controlled diabetes or prediabetes patients. Prediabetes state is rapidly increasing worldwide and is mainly associated with age and BMI. Controlling post-prandial glucose response in the overweight and obese population, also at risk for type-2 diabetes, appears to be key for preventing this disease.

Proteins are known to stimulate insulin secretion and a high protein diet has the potential to lower plasma glucose and fasting triglycerides in type-2 diabetic subjects (Van Loon LJ et al., 2000, Am J Clin Nutr 72:96-105; Gannon MC et al., 2003, Am J Clin Nutr 78:734-741). A further study published by Shertzer HG et al. (2011, J Nutr 141:582-587) revealed that dietary whey protein isolates administered to mice reduced the risk for metabolic disease and of developing diabetes associated with the consumption of a high-fat diet. WO2011/112695 discloses that health benefits provided by whey proteins include control of blood glucose such that they are suitable for diabetics. In recent studies consumption of whey protein isolate (25 to 50 g) 30 min before a meal has been to lower post-prandial glycaemia in healthy or type-2 diabetic subjects (Ma J., et al., Diabetes Res Clin Pract. 2015 May;108(2):e31-4; Jakubowicz D., et al., Diabetologia. 2014 Sep;57(9):1807-11). One study tested lower doses of whey protein isolate and reported that ingestion of 10 g of whey protein isolate 30 min before a meal could lower glucose excursion without changing insulin secretion in healthy adults (Akhavan T., et al., Am J Clin Nutr. 2010 Apr; 91(4):966-75).

There is a persisting need in the food industry to further improve the nutritional solutions provided to diabetic subjects or subjects at risk for developing type-2 diabetes.

An object of the present invention is to improve the state of the art and to provide a new and better nutritional solution for improving the postprandial glucose profile in a subject.

### SUMMARY

As set forth in greater detail later herein, the inventor conducted a study that surprisingly and unexpectedly showed that whey protein micelles (WPM) administered prior to a meal provide a significant decrease in postprandial glucose compared to whey protein isolate (WPI). Also surprisingly, it was found that the intake of as small an amount of WPM as 10g WPM administered at about 30 or even about 10 minutes prior to the meal was sufficient to provide a significant reduction in post-prandial glucose, and was significantly more effective than the same amount of WPI.

Accordingly, in a non-limiting embodiment, the present invention provides a method of reducing postprandial glucose from a meal. The method comprises orally administering a composition comprising whey protein micelles (WPM) to an individual and then subsequently orally administering the meal to the individual after the oral administration of the composition comprising the WPM and within about one hour of the oral administration of the composition comprising the WPM, preferably at least about ten minutes after the oral administration of the composition comprising the WPM and within about one hour of the oral administration of the composition comprising the WPM, for instance at least about 10 minutes after the oral administration of the composition comprising the WPM and within about forty minutes after the oral administration of the composition comprising the WPM, such as about thirty minutes after the oral administration of the composition comprising the WPM.

In an aspect the present invention provides a composition for use in reducing postprandial glucose from a meal, the composition being orally administered to an individual that has the therapeutic condition obesity said composition comprising whey protein micelles (WPM); and subsequently orally administering the meal to the individual after the oral administration of the composition comprising the WPM and within about one hour of the oral administration of the composition comprising the WPM..

In a further aspect, the invention pertains to a non-therapeutic use of a composition comprising whey protein micelles (WPM), in one condition for which reduced postprandial glucose from a meal is beneficial, wherein said condition is overweight, comprising:
orally administering to an individual in need thereof or at risk thereof said composition comprising whey protein micelles (WPM); and
subsequently orally administering the meal to the individual after the oral administration of the composition comprising the WPM and within about one hour of the oral administration of the composition comprising the WPM.

In another aspect, the invention provides whey protein micelles for use in reducing postprandial glucose from a meal, in an individual that has obesity wherein the whey protein micelles (WPM) are provided to the individual within about one hour before the administration of the meal.

For example, the oral administration of the composition comprising the WPM can be from about ten minutes before the administration of the meal to about one hour before the administration of the meal, such as about ten minutes before the administration of the meal, such as about thirty minutes before the administration of the meal. Preferably the individual does not consume any food product other than optional water in a time period between (i) the administration of the composition comprising the WPM and (ii) the administration of the meal.

The composition can be administered to the individual in a serving that provides up to about 60 g WPM/serving, for example about 5 g to about 60 g WPM/serving, preferably about 5g to about 30g WPM/serving, such as about 10 g to about 30 g WPM/serving, preferably about 5g to about 15g WPM/serving, such as about 10 g WPM/serving.

In an embodiment, the postprandial glucose achieved by the administration of the composition comprising the WPM before the administration of the meal (e.g., administration of the WPM about ten or about thirty minutes before the meal) is lower than postprandial glucose achieved by the administration of the same amount of whey protein in a form other than as whey protein micelles, e.g. as whey protein isolate, the same amount of time before the meal.

The composition comprising the WPM can be an oral nutritional supplement (ONS) that provides incomplete nutrition. Optionally the composition can comprise one or more ingredients additional to WPM, for example an optional additional component selected from the group consisting of a protein, a carbohydrate, a lipid, a vitamin, a mineral, excipients, emulsifiers, stabilizers, and mixtures thereof. The final formulation can be in a liquid or gel format ready to consumed, or in a powder format to be reconstituted in water before use.

The present disclosure provides a method of treating or preventing at least one condition for which reduced postprandial glucose is beneficial. The method comprises orally administering a composition comprising WPM to an individual in need thereof or at risk thereof and then subsequently orally administering a meal to the individual after the oral administration of the composition comprising the WPM and within about one hour of the oral administration of the composition comprising the WPM, preferably at least about ten minutes after the oral administration of the composition comprising the WPM and within about one hour of the oral administration of the composition comprising the WPM, for example at least about ten minutes after the oral administration of the composition comprising the WPM and within about forty minutes after the oral administration of the composition comprising the WPM, for example from about ten minutes after the oral administration of the composition comprising the WPM to about thirty minutes after the oral administration of the composition comprising the WPM, such as about ten, about twenty, or about thirty minutes after the oral administration of the composition comprising the WPM. In an embodiment the method comprises orally administering a composition comprising WPM to an individual in need thereof or at risk thereof and then subsequently orally administering a meal to the individual about ten minutes after the oral administration of the composition comprising the WPM. In another embodiment the method comprises orally administering a composition comprising WPM to an individual in need thereof or at risk thereof and then subsequently orally administering a meal to the individual about thirty minutes after the oral administration of the composition comprising the WPM.

Advantageously whey protein micelles (WPM) administered prior to a meal according to the present invention provide a significant decrease in postprandial glucose compared to whey protein isolate (WPI).

Further, surprisingly, intake of as small an amount of WPM as 10g WPM administered at about 30 or even about 10 minutes prior to the meal has been shown to be sufficient to provide a significant reduction in post-prandial glucose, and was significantly more effective than the same amount of WPI. Further, advantageously this makes it possible to achieve significant effects on postprandial glucose with a relatively small amount of protein administered at a relatively short time period before a regular meal. This provides numerous advantages such as permitting the pre-meal composition to be relatively low in protein and calories, making it possible to provide the pre-meal composition in the context of a calorie and/or protein restricted nutrition for overweight, obese, pre-diabetic or Type-2 diabetic individuals. Also this facilitates the provision of the pre-meal protein composition in a relatively low volume making it convenient to use.

Additional features and advantages are described in, and will be apparent from, the following Detailed Description and the Figures.

### BRIEF DESCRIPTION OF DRAWINGS

**FIG. 1** is a graph showing glucose excursion (over baseline) over time when WPM or WPI are consumed 30 min before a meal
**FIG. 2** is a graph showing glucose excursion (over baseline) over time when WPM or WPI are consumed 10 min before a meal

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages are by weight of the total weight of the composition unless expressed otherwise. Similarly, all ratios are by weight unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth. Ranges defined using "between" include the referenced endpoints.

As used herein and in the appended claims, the singular form of a word includes the plural, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y." Similarly, "at least one of X or Y" should be interpreted as "X," or "Y," or "both X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of" and "consisting of" the disclosed components. "Consisting essentially of" means that the embodiment or component thereof comprises more than 50 wt.% of the individually identified components, preferably at least 75 wt.% of the individually identified components, more preferably at least 85 wt.% of the individually identified components, most preferably at least 95 wt.% of the individually identified components, for example at least 99 wt.% of the individually identified components.

Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

"Animal" includes, but is not limited to, mammals, which includes but is not limited to rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage, e.g., an animal benefitting from reduced postprandial glucose. While the term "individual" or "subject" is often used herein to refer to a human, the present disclosure is not so limited. Accordingly, the term "individual" or "subject" refers to any animal, mammal or human that can benefit from the methods and compositions disclosed herein.

The relative terms "improved," "reduced," "enhanced" and the like refer to the effects of the method disclosed herein on postprandial glucose, particularly the administration of a composition containing WPM before administration of a meal (e.g., about thirty minutes before the meal), relative to administration of an identically formulated meal but administered without the WPM (e.g., no WPM within about one hour of the meal) or relative to administration of an identically formulated meal concurrently with the WPM (i.e., at approximately the same time).

As used herein, the terms "treat" and "treatment" mean to administer a composition as disclosed herein to a subject having a condition in order to lessen, reduce or improve at least one symptom associated with the condition and/or to slow down, reduce or block the progression of the condition. The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

The terms "prevent" and "prevention" mean to administer a composition as disclosed herein to a subject is not showing any symptoms of the condition to reduce or prevent development of at least one symptom associated with the condition. Furthermore, "prevention" includes reduction of risk, incidence and/or severity of a condition or disorder. As used herein, an "effective amount" is an amount that treats or prevents a deficiency, treats or prevents a disease or medical condition in an individual, or, more generally, reduces symptoms, manages progression of the disease, or provides a nutritional, physiological, or medical benefit to the individual.

"Overweight" is defined for a human as a body mass index (BMI) between 25 and 30 kg/m². "Obese" is defined for a human as a BMI of at least 30 kg/m², for example 30-39.9 kg/m².

"Diabetes" encompasses both the type I and type II forms of the disease. Non-limiting examples of risk factors for diabetes include: waistline of more than 40 inches for men or 35 inches for women, blood pressure of 130/85 mmHg or higher, triglycerides above 150 mg/dl, fasting blood glucose greater than 100 mg/dl or high-density lipoprotein of less than 40 mg/dl in men or 50 mg/dl in women. Therefore, an "individual at risk of diabetes" may have one or more of these factors present.

"Pre-diabetes" means that the individual has at least one of the following characteristics: a glycated hemoglobin (A1C) level between 5.7 and 6.4 percent, a fasting blood glucose from 100 to 125 mg/dL (5.6 to 7.0 mmol/L), or a blood sugar level from 140 to 199 mg/dL (7.8 to 11.0 mmol/L).

As used herein, "administering" includes another person providing a referenced composition to an individual so that the individual can consume the composition and also includes merely the act of the individual themselves consuming a referenced composition.

The terms "food," "food product" and "food composition" mean a composition that is intended for ingestion by an individual, such as a human, and that provides at least one nutrient to the individual. "Food" and its related terms include any food, feed, snack, food supplement, treat, meal substitute, or meal replacement, whether intended for a human or an animal. Animal food includes food or feed intended for any domesticated or wild species. In preferred embodiments, a food for an animal represents a pelleted, extruded, or dry food, for example, extruded pet foods such as foods for dogs and cats.

The terms "serving" or "unit dosage form," as used herein, are interchangeable and refer to physically discrete units suitable as unitary dosages for human and animal subjects, each unit containing a predetermined quantity of the composition comprising WPM disclosed herein in an amount sufficient to produce the desired effect, preferably in association with a pharmaceutically acceptable diluent, carrier or vehicle. The specifications for the unit dosage form depend on the particular compounds employed, the effect to be achieved, and the pharmacodynamics associated with each compound in the host. In an embodiment, the unit dosage form can be a predetermined amount of liquid housed within a container such as a bottle.

An "oral nutrition supplement" or "ONS" is a composition comprising at least one macronutrient and/or at least one micro nutrient, for example in a form of sterile liquids, semi-solids or powders, and intended to supplement other nutritional intake such as that from food. In some embodiments, an ONS can be a beverage in liquid form that can be consumed without further addition of liquid, for example an amount of the liquid that is one serving of the composition.

As used herein, "incomplete nutrition" refers to preferably nutritional products that do not contain sufficient levels of macronutrients (protein, fats and carbohydrates) or micronutrients to be sufficient to be a sole source of nutrition for the animal to which the nutritional product is being administered.

"Whey protein micelles" are defined herein as described in WO2007/110411 A2. Particularly, the "whey protein micelles" are the micelles comprised in the whey protein micelles concentrate obtainable by the process as disclosed in WO2007/110411 A2. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, in the concentrate, the powder and reconstituted from the powder for example in water. The "whey protein micelles" are physically stable in dispersion, as powder as well as during spray-drying or freeze-drying.

### Embodiments

An aspect of the present disclosure is a composition for use in reducing postprandial glucose from a meal, the composition orally administered to an individual that has the therapeutic condition obesity said composition comprising whey protein micelles (WPM); and subsequently orally administered the meal to the individual after the oral administration of the composition comprising the WPM and within about one hour of the oral administration of the composition comprising the WPM.

In a further aspect, the invention pertains to a non-therapeutic use of a composition comprising whey protein micelles (WPM), in one condition for which reduced postprandial glucose from a meal is beneficial, wherein said condition is overweight, comprising:
orally administering to an individual in need thereof or at risk thereof said composition comprising whey protein micelles (WPM); and
subsequently orally administering the meal to the individual after the oral administration of the composition comprising the WPM and within about one hour of the oral administration of the composition comprising the WPM.

In another aspect, the invention provides whey protein micelles for use in reducing postprandial glucose from a meal, in an individual that has obesity wherein the whey protein micelles (WPM) are provided to the individual within about one hour before the administration of the meal.

The method comprises orally administering a composition comprising whey protein micelles (WPM) to an individual and then subsequently orally administering the meal to the individual after the oral administration of the composition comprising the WPM and within about one hour of the oral administration of the composition comprising the WPM, preferably at least about ten minutes after the oral administration of the composition comprising the WPM and within about one hour of the oral administration of the composition comprising the WPM, more preferably at least about ten minutes after the oral administration of the composition comprising the WPM and within about forty minutes of the oral administration of the composition comprising the WPM, for example about ten, about twenty, or about thirty minutes after the oral administration of the composition comprising the WPM. As used herein, "subsequently" means at least about five minutes later, preferably at least about ten minutes later.

The present disclosure provides a method of treating or preventing at least one condition for which reduced postprandial glucose (PPG) is beneficial. The method comprises orally administering a composition comprising WPM to an individual in need thereof or at risk thereof and then subsequently orally administering a meal to the individual after the oral administration of the composition comprising the WPM and within about one hour of the oral administration of the composition comprising the WPM, preferably at least about ten minutes after the oral administration of the composition comprising the WPM and within about one hour of the oral administration of the composition comprising the WPM, more preferably at least about ten minutes after the oral administration of the composition comprising the WPM and within about forty minutes of the oral administration of the composition comprising the WPM, for example about ten minutes, about twenty minutes or about thirty minutes after the oral administration of the composition comprising the WPM. In a preferred embodiment, the composition comprising WPM is orally administered to an individual about ten minutes before a meal. In another preferred embodiment, the composition comprising WPM is orally administered to an individual about thirty minutes before a meal.

As used herein, "meal" refers to one or more food products consumed at substantially the same time as each other; preferably such that at least one macronutrient and at least one micronutrient are provided by consuming the meal; more preferably such that one or more proteins, one or more carbohydrates, one or more fats, one or more vitamins and one or more minerals are provided by consuming the meal. Preferably the meal comprises a plurality of food products. In an embodiment, the meal provides 200 kcal to 1,000 kcal to the individual, preferably 250 kcal to 900 kcal, more preferably 300 kcal to 850 kcal, and most preferably 350 kcal to 800 kcal. In an embodiment, the meal is substantially free of WPM (i.e., less than 2.5 wt.%, preferably less than 2.0 wt.%, more preferably less than 1.0 wt.%, most preferably less than 0.5 wt.% WPM) or completely free of WPM. The meal can be any meal, for example breakfast, lunch or dinner.

The administration of the composition comprising the WPM can be between about ten minutes before the administration of the meal and about one hour before the administration of the meal, preferably from about ten minutes before the administration of the meal to about forty minutes before the administration of the meal, more preferably from about ten minutes before the administration of the meal to about thirty minutes before the administration of the meal, such as about ten minutes, about twenty minutes, or about thirty minutes before the administration of the meal. Preferably the individual does not consume any food product other than optional water in the time period between the administration of the composition comprising the WPM and the administration of the meal.

In some embodiments, the meal is breakfast. For example, the composition comprising the WPM can be administered to the individual before breakfast, and then the breakfast can be subsequently administered to the individual after the administration of the composition comprising WPM. For example, the breakfast can be administered between about ten minutes after the administration of the composition comprising WPM and about one hour after the administration of the composition comprising WPM, preferably at least about ten minutes after the oral administration of the composition comprising the WPM and within about forty minutes after the oral administration of the composition comprising the WPM, preferably from about ten minutes after the oral administration of the composition comprising the WPM to about thirty minutes after the oral administration of the composition comprising the WPM, more for example about thirty minutes after the administration of the composition comprising WPM.

As used herein, "breakfast" is the first meal consumed by the individual on the particular day. For example, breakfast can be consumed before noon according to the local time of the individual, preferably before 11:00 AM according to the local time of the individual, more preferably before 10:00 AM according to the local time of the individual, most preferably before 9:00 AM according to the local time of the individual, but after the individual has awakened from sleep and/or after 4:00 AM according to the local time of the individual, preferably after 5:00 AM according to the local time of the individual, more preferably after 6:00 AM according to the local time of the individual, most preferably after 7:00 AM according to the local time of the individual.

In an embodiment, the postprandial glucose achieved by the administration of the composition comprising the WPM before the administration of the meal (e.g., administration of the WPM about ten minutes before the meal, e.g. about thirty minutes before the meal) is lower than postprandial glucose from administration of an identically formulated composition comprising whey protein in a non-micellar form, e.g. in the form of whey protein isolate (WPI) at the same amount of time before the administration of the meal.

In an embodiment, the composition is administered to an individual in a serving that provides at least about 5 g WPM, such as at least about 10 g WPM. In some embodiments, up to 30 g WPM are administered per serving of the composition.

In a preferred embodiment, the composition comprising the WPM is provided in the form of a liquid drink, and optionally may further comprise one or more of lipid, carbohydrate or another source of protein, and optionally further comprises a dietary flavoring (e.g., vanilla).

In some embodiments, the composition comprising WPM may be in the form of a nutritional composition or a nutritional supplement. The term "nutritional supplement" refers to a product which is intended to supplement the general diet of a subject. For example, the composition comprising the WPM can be an oral nutritional supplement (ONS) that provides incomplete nutrition. The ONS can comprise one or more ingredients additional to WPM, for example an additional component selected from the group consisting of a protein, a carbohydrate, a lipid, a vitamin, a mineral, and mixtures thereof.

Non-limiting examples of suitable proteins additional to the WPM include animal proteins, such as milk protein, meat protein and egg protein; or vegetable proteins, such as soy protein, wheat protein, rice protein, pea protein, corn protein, canola protein, oat protein, potato protein, peanut protein, and any proteins derived from beans, buckwheat or lentils. Milk proteins, such as casein and whey, and soy proteins may be preferred for some applications. If the protein is a milk protein or a milk protein fraction, the protein may be, for example, sweet whey, acid whey, α-lactalbumin, β-lactoglobulin, bovine serum albumin, acid casein, caseinates, α-casein, β-casein and/or γ-casein.

Non-limiting examples of suitable carbohydrates include mono-saccharides and/or disaccharides, slowly digested fully caloric carbohydrates, oligosaccharides, or mixtures thereof. Particular non-limiting examples include maltodextrin, maltose, high-maltose corn syrup, fructose, galactose, sucrose, lactose or a mixture of thereof.

Non-limiting examples of suitable lipids include monoacylglycerols (MAG), diacylglycerol (DAG), long chain triglycerides (LCT), medium chain triglycerides (MCT), short chain fatty acids (SCFA), branched chain fatty acids (BCFA), structured MAG, structured DAG, fatty acids (free and/or bound, e.g., esterified to glycerol or as ethyl esters), phospholipids, lyso-phospholipids, sphingomyelin, gangliosides, specialized pro-resolving mediators (SPMs), or mixtures thereof. The fatty acids that are free and/or bound may include one or more of linoleic acid (18:2n-6), alpha-linolenic acid (18:3n-3), dihomogammalinolenic acid (20:3n-6), gamma-linolenic acid (GLA, 18:3n-6), stearidonic acid (18:4n-3), docosapentaenoic acid (DPA, 22:5n-3) or mixtures thereof. The source of the lipids may be one or more of animal, plant, fermented, microalgae, GMO, non-GMO or mixtures thereof.

An embodiment of the composition comprising the WPM is a non-complete liquid ONS that can further comprise protein, e.g., milk protein concentrate. In an embodiment of this non-complete liquid ONS, the composition can consist essentially of water, the WPM and the protein (e.g., milk protein concentrate) and optionally a flavoring. Preferably the non-complete liquid ONS is a "shot," for example having a volume of about 30 mL to about 300 mL, preferably about 40 mL to about 200 mL, more preferably about 50 mL to about 150mL, for example about 100 mL. Preferably the non-complete liquid ONS is in a unit dosage form that provides at least about 5 g WPM, such as at least about 10 g WPM, and in some embodiments, no greater than 60g WPM, preferably no greater than 30 g WPM..

The format of the WPM product can contain excipients, emulsifiers, stabilizers and mixtures thereof, and the final formulation can be in a liquid format ready to be consumed, or in a powder format to be reconstituted in water before use.

The WPM can be about 10 to about 200 g/L of a liquid composition. Additional proteins can be 0 to about 120 g/L of the composition. The lipids can be 0 to about 120 g/L of the composition, preferably about 10 g/L to about 200 g/L of the composition. The carbohydrates can be 0 to about 200 g/L of the composition, preferably about 10 g/L to about 200 g/L of the composition.

The composition comprising the WPM may further comprise one or more additional components such as minerals; vitamins; salts; or functional additives including, for example, palatants, colorants, emulsifiers, antimicrobial or other preservatives. Non-limiting examples of suitable minerals for the compositions disclosed herein include calcium, phosphorous, potassium, sodium, iron, chloride, boron, copper, zinc, magnesium, manganese, iodine, selenium, chromium, molybdenum, fluoride and any combination thereof. Non-limiting examples of suitable vitamins for the compositions disclosed herein include water-soluble vitamins (such as thiamin (vitamin B1), riboflavin (vitamin B2), niacin (vitamin B3), pantothenic acid (vitamin B5), pyridoxine (vitamin B6), biotin (vitamin B7), myo-inositol (vitamin B8) folic acid (vitamin B9), cobalamin (vitamin B12), and vitamin C) and fat-soluble vitamins (such as vitamin A, vitamin D, vitamin E, and vitamin K) including salts, esters or derivatives thereof. Inulin, taurine, carnitine, amino acids, enzymes, coenzymes, and any combination thereof may be included in various embodiments.

The individual may be a mammal such as a human, canine, feline, equine, caprine, bovine, ovine, porcine, cervine or a primate. Preferably the individual is a human.

All references herein to treatment include curative, palliative and prophylactic treatment. Treatment may also include arresting progression in the severity of a disease. Both human and veterinary treatments are within the scope of the present disclosure. Preferably the composition comprising the WPM is administered in a serving or unit dosage form that provides a therapeutically effective or prophylactically effective amount of the WPM.

In view of the above, disclosed herein is a method of reducing postprandial glucose from a meal, the method comprising: orally administering to an individual a composition comprising whey protein micelles (WPM); and subsequently orally administering the meal to the individual after the oral administration of the composition comprising the WPM and within about one hour of the oral administration of the composition comprising the WPM.

The postprandial glucose in the individual is preferably reduced relative to postprandial glucose from oral administration of a composition comprising the same amount of whey protein in a non-micellar form, e.g. in the form of whey protein isolate (WPI) at the same amount of time before the administration of the meal.

The individual preferably has obesity.

The composition comprising the WPM is preferably a liquid. The composition can preferably be administered to the individual in a serving comprising at least about 5.0 g of the WPM up to about 60.0 g of the WPM, preferably about 5.0g of the WPM up to about 30.0g of the WPM, such as about 10.0 g of the WPM up to about 30.0 g of the WPM. The composition can comprise the WPM at least one additional component selected from the group consisting of another protein source, a lipid and a carbohydrate.

The meal can be administered between about ten minutes after the administration of the composition comprising the WPM and about one hour after the administration of the composition comprising the WPM, preferably from about ten minutes after the administration of the composition comprising the WPM to about forty minutes after the administration of the composition comprising the WPM, such as from about ten minutes after the administration of the composition comprising the WPM to about thirty minutes after the administration of the composition comprising the WPM. For example, the meal can be administered about ten minutes, about twenty minutes, or about thirty minutes after the administration of the composition comprising the WPM.

The meal can be breakfast. The composition can be a liquid incomplete nutrition oral nutritional supplement (ONS).

Preferably, the individual does not consume any food products other than optional water during a time period from the oral administration of the composition comprising the WPM to the individual to the oral administration of the meal to the individual.

Disclosed herein is a method of treating or preventing a condition for which reduced postprandial glucose from a meal is beneficial, the method comprising: orally administering to the individual a composition comprising whey protein micelles (WPM); and subsequently orally administering the meal to the individual after the oral administration of the composition comprising the WPM and within about one hour of the oral administration of the composition comprising the WPM, preferably at least about ten minutes after the oral administration of the composition comprising the WPM and within about one hour of the oral administration of the composition comprising the WPM.

In another embodiment the invention pertains to whey protein micelles for use in the treatment and/or prevention of a disorder linked to an increase in plasma postprandial glucose concentration in an individual in need thereof or at risk thereof, wherein the WPM are provided prior to a regular meal, preferably at least 10 minutes before the meal and within about one hour before the meal, more preferably at least 10 minutes before the meal and within about forty minutes before the meal, for example about ten minutes, about twenty minutes or about thirty minutes before the meal.

In a preferred embodiment, the whey protein micelles are for use in an obese patient.

In a further aspect, the invention pertains to the use of whey protein micelles to decrease plasma postprandial glucose concentration in an individual, wherein the WPM are provided prior to a regular meal, preferably at least 10 minutes before the meal and within about one hour before the meal, more preferably at least 10 minutes before the meal and within about forty minutes before the meal, for example about ten minutes, about twenty minutes or about thirty minutes before the meal.

In an embodiment, the meal can be administered from about ten minutes after the administration of the composition comprising the WPM to about forty minutes after the administration of the composition comprising the WPM. For example, the meal can be administered about ten minutes after the administration of the composition comprising the WPM. In another embodiment the meal can be administered about thirty minutes after the administration of the composition comprising the WPM.

In an embodiment, the WPM is administered to an individual in a serving that provides at least about 5 g WPM, such as at least about 10 g WPM. In some embodiments, up to 60 g WPM are administered per serving of the composition. In some embodiments, up to 30 g WPM are administered per serving of the composition. In one preferred embodiment about 5g to about 15g of WPM, such as about 10g of WPM are provided per serving of the composition.

### EXAMPLE

The following non-limiting example presents clinical data developing and supporting the concepts of the present invention.

The effects of whey protein administration prior to a meal on post-prandial glucose was tested in overweight/obese subjects in a crossover trial. Solutions (100 ml) containing either water alone (control), 10 g of whey protein isolate (WPI) or 10g of whey protein micelles (WPM) (produced according to WO2007/11041) were consumed 10 or 30 min before a standard meal. Subcutaneous interstitial glucose concentration was measured before (fasting glucose) and after consumption of the whey protein and the standard meal (up to 120 min) using a continuous glucose monitoring system (FreeStyle libre^{®}, Abbott).

The standard meal consumed by the test subjects was a breakfast consisting of two slices of white bread, jam and a glass of orange juice (calculated calories 320 kcal).

The study population was 14 overweight obese adult males and females (8 women, 6 men), BMI 31.2 +/- 2.8, age 49 +/-8.

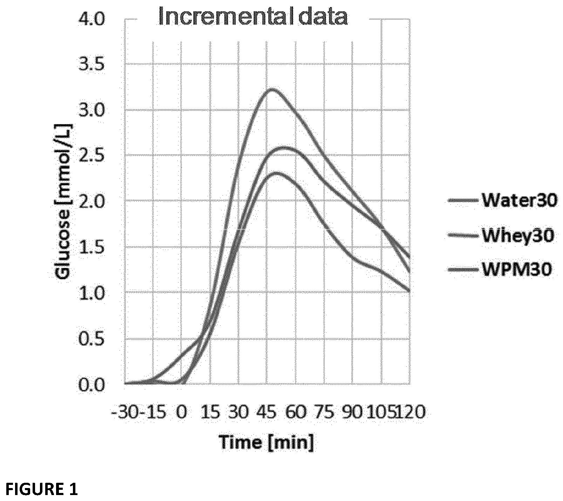

Figure 1 illustrates the change in interstitial glucose level (average of 14 subjects) over time with WPM, WPI or water (control) administered 30 minutes before a standard meal. Time 0 correspond to ingestion of the standard meal (breakfast).

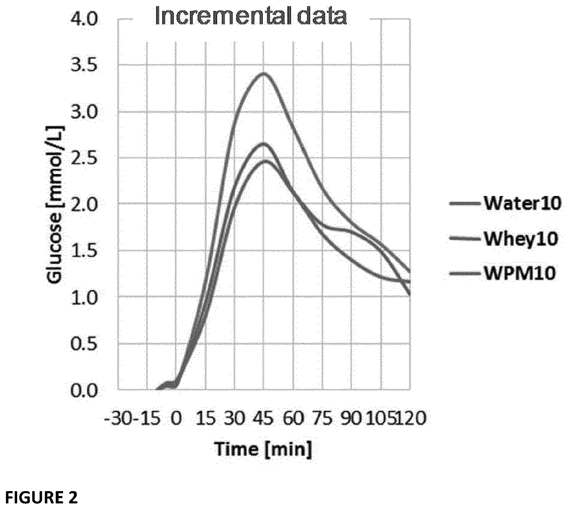

Figure 2 illustrates the change in interstitial glucose level (average of 14 subjects) over time with WPM, WPI or water (control) administered 10 minutes before a standard meal. Time 0 correspond to ingestion of the standard meal (breakfast).

When taken 30 min before the standard breakfast, both WPM and WPI reduced significantly iCmax of the postprandial glucose curve compared with the control (WPI: - 0.7 mM, p = 0.02; WPM: - 1.1 mM, p < 0.01). WPM administered 30 minutes before the meal also decreased significantly postprandial glucose iAUC, whereas only a trend for lowering iAUC was observed with WPI (WPI: - 14%, p= 0.1; WPM: - 30%, p < 0.01). Surprisingly, postprandial glucose iAUC with WPM administered 30 minutes prior to the meal was significantly lower than the one observed with WPI (-19%, p = 0.04).

Similarly, when WPM and WPI were consumed 10 min before the standard meal glucose iCmax were significantly lower than after water consumption (WPI: - 0.9 mM, p = 0.01; WPM: - 1.1 mM, p < 0.01. Glucose iAUC was significantly decreased after WPM consumption and showed only a trend for reduction after WPI (WPI: - 18%, p = 0.08; WPM: - 25%, p = 0.02. Again, surprisingly, postprandial glucose iAUC with WPM administered 10 minutes prior to the meal was substantially lower than the one observed with WPI.

No significant difference was observed in glucose iCmax or iAUC between when the whey protein (WPM or WPI) was taken at 10 minutes compared to the same type of whey protein at 30 minutes before the meal respectively.

These results show that administration of WPM before a meal significantly reduce the postprandial glucose response (PPG) and demonstrate that WPM was more efficient than WPI to decrease post-prandial glucose when taken 10 minutes or 30 minutes before a meal. Further, these results that 10g of WPM administered 10 minutes or 30 minutes before a meal is sufficient to provide significant reduction in postprandial glucose.

These results also show that administration of WPM 10 min before a meal reduced post-prandial glucose at a similar extent than when consumed 30 min before.

## Claims

1. A composition for use in reducing postprandial glucose from a meal, the composition being orally administered to an individual that has the therapeutic condition obesity said composition comprising whey protein micelles (WPM); and subsequently orally administering the meal to the individual after the oral administration of the composition comprising the WPM and within about one hour of the oral administration of the composition comprising the WPM.

2. A non-therapeutic use of a composition comprising whey protein micelles (WPM), in one condition for which reduced postprandial glucose from a meal is beneficial, wherein said condition is overweight, comprising:
orally administering to an individual in need thereof or at risk thereof said composition comprising whey protein micelles (WPM); and
subsequently orally administering the meal to the individual after the oral administration of the composition comprising the WPM and within about one hour of the oral administration of the composition comprising the WPM.

3. The composition for use according to claim 1 or the non-therapeutic use of the composition according to claim 2 wherein the composition comprising the WPM is a liquid, such as a drink or an incomplete nutrition oral nutritional supplement (ONS).

4. The composition for use or the non-therapeutic use of the composition according to any one of claims 1 to 3 wherein the composition is administered to the individual in a serving comprising about 5.0 g of the WPM up to about 60.0 g of the WPM, preferably in a serving comprising about 5.0 g of the WPM up to about 30.0 g of the WPM, preferably in a serving comprising about 5.0 g of the WPM up to about 15.0 g of the WPM preferably in a serving comprising about 10.0g of the WPM.

5. The composition for use or the non-therapeutic use of the composition according to any one of claims 1 to 4 wherein the composition comprises the WPM and at least one additional component selected from the group consisting of a further protein, a lipid, a carbohydrate, a vitamin, a mineral and a flavour.

6. The composition for use or the non-therapeutic use of the composition according to any one of claims 1 to 5 wherein the meal is administered from about ten minutes after the administration of the composition comprising the WPM to about one hour after the administration of the composition comprising the WPM, preferably from about ten minutes after the administration of the composition comprising the WPM to about forty minutes after the administration of the composition comprising the WPM.

7. The composition for use or the non-therapeutic use of the composition according to any one of claims 1 to 6 wherein the meal is administered about thirty minutes after the administration of the composition comprising the WPM, or wherein the meal is administered about ten minutes after the administration of the composition comprising the WPM.

8. The composition for use or the non-therapeutic use of the composition according to any one of claims 1 to 7 wherein the meal is breakfast.

9. The composition for use or the non-therapeutic use of the composition according to any one of claims 1 to 8 wherein the postprandial glucose in the individual is reduced relative to postprandial glucose on administration of a composition comprising the same amount of whey protein in the form of a whey protein isolate, administered at same amount of time before the meal.

10. The composition for use or the non-therapeutic use of the composition according to claim 1 wherein the individual does not consume any food products other than optional water during a time period from the oral administration of the composition comprising the WPM to the individual to the oral administration of the meal to the individual.

11. A non-therapeutic use of whey protein micelles (WPM) for reducing postprandial glucose response in individual in need thereof or at risk thereof who is overweight, wherein the whey protein micelles (WPM) are provided to the individual within about one hour before the administration of a regular meal.

12. Whey protein micelles for use in reducing postprandial glucose from a meal, in an individual that has obesity wherein the whey protein micelles (WPM) are provided to the individual within about one hour before the administration of the meal.

13. The use of whey protein micelles according to claim 11 or the whey protein micelles for use according to any one of claim 12 wherein the meal is administered from about ten minutes after the administration of the WPM to about one hour after the administration of the WPM, preferably from about ten minutes after the administration of the WPM to about forty minutes after the administration the WPM.

14. The use of whey protein micelles or the whey protein micelles for use according to claim 13 wherein the meal is administered about thirty minutes after the administration of the WPM, or about ten minutes after the administration of the WPM.

15. The use of whey protein micelles or the whey protein micelles for use according to any one of claims 11 to 14 wherein the whey protein micelles are provided in a composition comprising about 5.0 g of the WPM up to about 60.0 g of the WPM per serving of the composition, preferably about 5.0 g of the WPM up to about 30.0 g of the WPM per serving of the composition, preferably about 5.0g to about 15.0g of the WPM per serving of the composition, such as about 10.0g of the WPM per serving of the composition.

16. The use of whey protein micelles or the whey protein micelles for use according to any one of claims 11 to 15 wherein the WPM are provided in the form of a liquid composition, such as a drink or an incomplete oral nutritional supplement (ONS).

17. The use of whey protein micelles or the whey protein micelles for use according to any one of claims 11 to 16 wherein the WPM are provided in the form of a liquid composition comprising the WPM and at least one additional component selected from the group consisting of a further protein, a lipid, a carbohydrate, a vitamin, a mineral and a flavour.

## Patentansprüche

1. Zusammensetzung zur Verwendung bei einem Reduzieren von postprandialer Glukose aus einer Mahlzeit, wobei die Zusammensetzung an eine Person oral verabreicht wird, die den therapeutischen Zustand Adipositas aufweist, die Zusammensetzung umfassend Molkenproteinmizellen (WPM); und anschließendes orales Verabreiche der Mahlzeit an die Person nach der oralen Verabreichung der Zusammensetzung, umfassend die WPM, und innerhalb von etwa einer Stunde nach der oralen Verabreichung der Zusammensetzung, umfassend die WPM.

2. Nicht therapeutische Verwendung einer Zusammensetzung, umfassend Molkenproteinmizellen (WPM), bei einem Zustand, für den reduzierte postprandiale Glukose aus einer Mahlzeit vorteilhaft ist, wobei der Zustand Übergewicht ist, umfassend:
orales Verabreichen an eine Person, die diese benötigt oder die ein Risiko dafür aufweist, der Zusammensetzung, umfassend Molkenproteinmizellen (WPM); und
anschließendes orales Verabreichen der Mahlzeit an die Person nach der oralen Verabreichung der Zusammensetzung, umfassend die WPM, und innerhalb von etwa einer Stunde nach der oralen Verabreichung der Zusammensetzung, umfassend die WPM.

3. Zusammensetzung zur Verwendung nach Anspruch 1 oder nicht therapeutische Verwendung der Zusammensetzung nach Anspruch 2, wobei die Zusammensetzung, umfassend die WPM, eine Flüssigkeit ist, wie etwa ein Getränk oder ein orales Nahrungsergänzungsmittel als unvollständige Ernährung (ONS).

4. Zusammensetzung zur Verwendung oder nicht therapeutische Verwendung der Zusammensetzung nach einem der Ansprüche 1 bis 3, wobei die Zusammensetzung der Person in einer Portion verabreicht wird, umfassend etwa 5,0 g der WPM bis etwa 60,0 g der WPM, vorzugsweise in einer Portion, umfassend etwa 5,0 g der WPM bis etwa 30,0 g der WPM, vorzugsweise in einer Portion, umfassend etwa 5,0 g der WPM bis etwa 15,0 g der WPM, vorzugsweise in einer Portion, umfassend etwa 10,0 g der WPM.

5. Zusammensetzung zur Verwendung oder nicht therapeutische Verwendung der Zusammensetzung nach einem der Ansprüche 1 bis 4, wobei die Zusammensetzung die WPM und mindestens eine zusätzliche Komponente umfasst, die aus der Gruppe ausgewählt ist, bestehend aus einem weiteren Protein, einem Lipid, einem Kohlenhydrat, einem Vitamin, einem Mineral und einem Geschmacksstoff.

6. Zusammensetzung zur Verwendung oder nicht therapeutische Verwendung der Zusammensetzung nach einem der Ansprüche 1 bis 5, wobei die Mahlzeit etwa zehn Minuten nach der Verabreichung der Zusammensetzung, umfassend die WPM, bis etwa eine Stunde nach der Verabreichung der Zusammensetzung, umfassend die WPM, vorzugsweise etwa zehn Minuten nach der Verabreichung der Zusammensetzung, umfassend die WPM, bis etwa vierzig Minuten nach der Verabreichung der Zusammensetzung, umfassend die WPM, verabreicht wird.

7. Zusammensetzung zur Verwendung oder nicht therapeutische Verwendung der Zusammensetzung nach einem der Ansprüche 1 bis 6, wobei die Mahlzeit etwa dreißig Minuten nach der Verabreichung der Zusammensetzung, umfassend die WPM, verabreicht wird oder wobei die Mahlzeit etwa zehn Minuten nach der Verabreichung der Zusammensetzung, umfassend die WPM, verabreicht wird.

8. Zusammensetzung zur Verwendung oder nicht therapeutische Verwendung der Zusammensetzung nach einem der Ansprüche 1 bis 7, wobei es sich bei der Mahlzeit um Frühstück handelt.

9. Zusammensetzung zur Verwendung oder nicht therapeutische Verwendung der Zusammensetzung nach einem der Ansprüche 1 bis 8, wobei die postprandiale Glukose der Person relativ zu der postprandialen Glukose bei der Verabreichung einer Zusammensetzung, umfassend die gleiche Menge Molkenprotein in der Form eines Molkenproteinisolats, die zu einem gleichen Zeitpunkt vor der Mahlzeit verabreicht wird, reduziert ist.

10. Zusammensetzung zur Verwendung nicht therapeutische Verwendung der Zusammensetzung nach Anspruch 1, wobei die Person während eines Zeitraums von der oralen Verabreichung der Zusammensetzung, umfassend die WPM, an die Person bis zu der oralen Verabreichung der Mahlzeit an die Person keine anderen Nahrungsmittel, außer optional Wasser, zu sich nimmt.

11. Nicht therapeutische Verwendung von Molkenproteinmizellen (WPM) zum Reduzieren einer postprandialen Glukoseantwort bei einer Person, die diese benötigt oder die das Risiko dafür aufweist, die übergewichtig ist, wobei die Molkenproteinmizellen (WPM) der Person innerhalb von etwa einer Stunde vor der Verabreichung einer regulären Mahlzeit bereitgestellt werden.

12. Molkenproteinmizellen zur Verwendung bei dem Reduzieren von postprandialer Glukose aus einer Mahlzeit bei einer Person, die Adipositas aufweist, wobei die Molkenproteinmizellen (WPM) der Person innerhalb von etwa einer Stunde vor der Verabreichung der Mahlzeit bereitgestellt werden.

13. Verwendung von Molkenproteinmizellen nach Anspruch 11 oder Molkenproteinmizellen zur Verwendung nach Anspruch 12, wobei die Mahlzeit etwa zehn Minuten nach der Verabreichung der WPM bis etwa eine Stunde nach der Verabreichung der WPM, vorzugsweise etwa zehn Minuten nach der Verabreichung der WPM bis etwa vierzig Minuten nach der Verabreichung der WPM, verabreicht wird.

14. Verwendung von Molkenproteinmizellen oder Molkenproteinmizellen zur Verwendung nach Anspruch 13, wobei die Mahlzeit etwa dreißig Minuten nach der Verabreichung der WPM oder etwa zehn Minuten nach der Verabreichung der WPM verabreicht wird.

15. Verwendung von Molkenproteinmizellen oder Molkenproteinmizellen zur Verwendung nach einem der Ansprüche 11 bis 14, wobei die Molkenproteinmizellen in einer Zusammensetzung bereitgestellt werden, umfassend etwa 5,0 g der WPM bis etwa 60,0 g der WPM pro Portion der Zusammensetzung, vorzugsweise etwa 5,0 g der WPM bis etwa 30,0 g der WPM pro Portion der Zusammensetzung, vorzugsweise etwa 5,0 g bis etwa 15,0 g der WPM pro Portion der Zusammensetzung, wie etwa 10,0 g der WPM pro Portion der Zusammensetzung.

16. Verwendung von Molkenproteinmizellen oder Molkenproteinmizellen zur Verwendung nach einem der Ansprüche 11 bis 15, wobei die WPM in der Form einer flüssigen Zusammensetzung, wie etwa eines Getränks oder eines unvollständigen oralen Nahrungsergänzungsmittels (ONS), bereitgestellt werden.

17. Verwendung von Molkenproteinmizellen oder Molkenproteinmizellen zur Verwendung nach einem der Ansprüche 11 bis 16, wobei die WPM in der Form einer flüssigen Zusammensetzung bereitgestellt werden, umfassend die WPM und mindestens eine zusätzliche Komponente, die aus der Gruppe ausgewählt ist, bestehend aus einem weiteren Protein, einem Lipid, einem Kohlenhydrat, einem Vitamin, einem Mineral und einem Geschmacksstoff.

## Revendications

1. Composition pour utilisation dans la réduction de la glycémie postprandiale d'un repas, la composition étant administrée par voie orale à un individu qui souffre de la condition thérapeutique d'obésité, ladite composition comprenant des micelles de protéine de lactosérum (WPM) ; et l'administration par voie orale ultérieure du repas à l'individu après l'administration orale de la composition comprenant les WPM et dans un délai d'environ une heure après l'administration orale de la composition comprenant les WPM.

2. Utilisation non thérapeutique d'une composition comprenant des micelles de protéines de lactosérum (WPM), dans un état pour lequel la réduction de la glycémie postprandiale d'un repas est bénéfique, dans laquelle ledit état est un surpoids, comprenant :
l'administration par voie orale à un individu qui en a besoin ou présentant un risque de ladite composition comprenant des micelles de protéines de lactosérum (WPM) ; et
l'administration par voie orale ultérieure du repas à l'individu après l'administration orale de la composition comprenant les WPM et dans un délai d'environ une heure après l'administration orale de la composition comprenant les WPM.

3. Composition pour utilisation selon la revendication 1 ou utilisation non thérapeutique de la composition selon la revendication 2, dans laquelle la composition comprenant les WPM est un liquide, tel qu'une boisson ou un complément nutritionnel oral (CNO) de nutrition incomplète.

4. Composition pour utilisation ou utilisation non thérapeutique de la composition selon l'une quelconque des revendications 1 à 3, dans laquelle la composition est administrée à l'individu dans une portion comprenant environ 5,0 g de WPM jusqu'à environ 60,0 g de WPM, de préférence dans une portion comprenant environ 5,0 g de WPM jusqu'à environ 30,0 g de WPM, de préférence dans une portion comprenant environ 5,0 g de WPM jusqu'à environ 15,0 g de WPM, de préférence dans une portion comprenant environ 10,0 g de WPM.

5. Composition pour utilisation ou utilisation non thérapeutique de la composition selon l'une quelconque des revendications 1 à 4, dans laquelle la composition comprend les WPM et au moins un composant supplémentaire choisi dans le groupe constitué d'une autre protéine, d'un lipide, d'un glucide, d'une vitamine, d'un minéral et d'un arôme.

6. Composition pour utilisation ou utilisation non thérapeutique de la composition selon l'une quelconque des revendications 1 à 5, dans laquelle le repas est administré entre environ dix minutes après l'administration de la composition comprenant les WPM et environ une heure après l'administration de la composition comprenant les WPM, de préférence entre environ dix minutes après l'administration de la composition comprenant les WPM et environ quarante minutes après l'administration de la composition comprenant les WPM.

7. Composition pour utilisation ou utilisation non thérapeutique de la composition selon l'une quelconque des revendications 1 à 6, dans laquelle le repas est administré environ trente minutes après l'administration de la composition comprenant les WPM, ou dans laquelle le repas est administré environ dix minutes après l'administration de la composition comprenant les WPM.

8. Composition pour utilisation ou utilisation non thérapeutique de la composition selon l'une quelconque des revendications 1 à 7, dans laquelle le repas est le petit-déjeuner.

9. Composition pour utilisation ou utilisation non thérapeutique de la composition selon l'une quelconque des revendications 1 à 8, dans laquelle la glycémie postprandiale chez l'individu est réduite par rapport à la glycémie postprandiale lors de l'administration d'une composition comprenant la même quantité de protéines de lactosérum sous forme d'isolat de protéines de lactosérum, administrée dans le même laps de temps avant le repas.

10. Composition pour utilisation ou utilisation non thérapeutique de la composition selon la revendication 1, dans laquelle l'individu ne consomme aucun produit alimentaire autre que de l'eau facultative pendant une période de temps allant de l'administration orale de la composition comprenant les WPM à l'individu à l'administration orale du repas à l'individu.

11. Utilisation non thérapeutique de micelles de protéines de lactosérum (WPM) destinée à réduire la réponse de glycémie postprandiale chez un individu qui en a besoin ou qui présente un risque et qui est en surpoids, dans laquelle les micelles de protéines de lactosérum (WPM) sont fournies à la personne dans l'heure qui précède l'administration d'un repas normal.

12. Micelles de protéines de lactosérum pour utilisation dans la réduction de glycémie postprandiale d'un repas, chez un individu qui souffre d'obésité, dans laquelle les micelles de protéines de lactosérum (WPM) sont fournies à l'individu environ une heure avant l'administration du repas.

13. Utilisation de micelles de protéines de lactosérum selon la revendication 11 ou micelles de protéines de lactosérum pour utilisation selon la revendication 12, dans lesquelles le repas est administré entre environ dix minutes après l'administration des WPM et environ une heure après l'administration des WPM, de préférence entre environ dix minutes après l'administration des WPM et environ quarante minutes après l'administration des WPM.

14. Utilisation de micelles de protéines de lactosérum ou micelles de protéines de lactosérum pour utilisation selon la revendication 13, dans lesquelles le repas est administré environ trente minutes après l'administration des WPM, ou environ dix minutes après l'administration des WPM.

15. Utilisation de micelles de protéines de lactosérum ou micelles de protéines de lactosérum pour utilisation selon l'une quelconque des revendications 11 à 14, dans lesquelles les micelles de protéines de lactosérum sont fournies dans une composition comprenant environ 5,0 g de WPM jusqu'à environ 60,0 g de WPM par portion de la composition, de préférence environ 5,0 g de WPM jusqu'à environ 30,0 g de WPM par portion de la composition, de préférence environ 5,0 g à environ 15,0 g de WPM par portion de la composition, par exemple environ 10,0 g de WPM par portion de la composition.

16. Utilisation de micelles de protéines de lactosérum ou micelles de protéines de lactosérum pour utilisation selon l'une quelconque des revendications 11 à 15, dans lesquelles les WPM sont fournies sous la forme d'une composition liquide, telle qu'une boisson ou un complément nutritionnel oral (CNO) incomplet.

17. Utilisation de micelles de protéines de lactosérum ou micelles de protéines de lactosérum pour utilisation selon l'une quelconque des revendications 11 à 16, dans lesquelles les WPM sont fournies sous la forme d'une composition liquide comprenant les WPM et au moins un composant supplémentaire choisi dans le groupe constitué d'une autre protéine, d'un lipide, d'un glucide, d'une vitamine, d'un minéral et d'un arôme.
